(19) Europäisches Patentamt
European Patent Office
Office européen des brevets

(11) **EP 2 674 193 A1**

(12) **EUROPEAN PATENT APPLICATION**

(43) Date of publication:
**18.12.2013 Bulletin 2013/51**

(51) Int Cl.:
*A61N 1/37* (2006.01)  *A61N 1/36* (2006.01)
*G01R 33/28* (2006.01)

(21) Application number: **12171506.4**

(22) Date of filing: **11.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Sapiens Steering Brain Stimulation B.V.**
**5656 AE Eindhoven (NL)**

(72) Inventors:
• **Tol, Jeroen Jacob Arnold**
**5645 JG Eindhoven (NL)**
• **Budzelaar, Franciscus Paulus Maria**
**5632 PL Eindhoven (NL)**

(74) Representative: **Rupprecht, Kay**
**Meissner, Bolte & Partner GbR**
**Widenmayerstraße 47-50**
**80538 München (DE)**

(54) **Synchronization of the operation of an implantable medical device with the application of RF and/or gradient fields in an MRI system**

(57) The present invention relates to an electronic system (10) of an implantable medical device, comprising at least one first circuit (20), at least one tracking means, which is configured such that radio frequency bursts and/or a gradient magnetic field of a magnetic resonance imaging apparatus can be tracked, and at least one synchronizing means, whereby the synchronizing means is configured such that based on the tracked radio frequency bursts and/or the gradient magnetic field the at least one first circuit (20) and/or the electronic system (10) as a whole is synchronized with the radio frequency bursts and/or the gradient magnetic field.

FIG. 2a

EP 2 674 193 A1

FIG. 2b

## Description

The present invention relates to an electronic system, especially an electronic system for a medical device and to a medical system.

More and more implants require that the patient carrying such a device might undergo an MRI scan during the lifetime of the implant. This not only implies that the device needs to be MRI safe but also that the device survives an MRI scan. Moreover, if the implant cannot be switched off (completely) during an MRI scan for therapeutic reasons or simply because it cannot to be turned on afterwards if one does so, its electronics needs to be designed in such a way that it does not interfere with the picture making process of an MRI machine.

Almost all active electronic devices such as implantables generate high frequency signals.

In particular the digital parts of an implantable, such as microprocessors or digital communication lines, generate square waves that contain harmonic components that are many times the applied clock frequency. MRI scanners can be viewed as extremely sensitive radio receivers for signals around their RF operating frequency, which is approximately 64 MHz for a 1.5 T scanner and 128 MHz for a 3 T scanner. The emitted harmonics which fall in those frequency bands may easily interfere with the imaging process of an MRI machine leading to unusable images.

US 7,660,620 B2 discloses timing techniques for magnetic resonance imaging. Timing information is received from an implantable medical device and there are means for synchronizing application of electromagnetic radiation with the received timing information and means for imaging cardiac tissue during application of the cardiac stimulation by the implantable medical device based upon the timing information.

US 2010/0106006 A1 discloses a device comprising a telemetry unit to receive information from an implantable medical device, a control unit that defines timing of electromagnetic radiation bursts based on information received from the implantable medical device, and an electromagnetic radiation source that applies electromagnetic radiation bursts to a patient within which the implantable medical device is implanted based on the defined timing. It is possible that information regarding the timing of the electromagnetic radiation bursts is sent to the implantable medical device to control the operation of the implantable medical device.

It is therefore an object of the present invention to improve an electronic system for a medical device, particularly in that the electronics of an implant are configured such that it will not interfere with the MRI imaging process without switching off the electronics of the implant.

The above object is solved by an electronic system according to the present invention with the features of claim 1. Accordingly, an electronic system is provided, especially an electronic system for a medical device, comprising at least one first circuit, at least one tracking means, which is configured such that radio frequency bursts and/or a gradient magnetic field of a magnetic resonance imaging apparatus can be tracked, and at least one synchronizing means, whereby the synchronizing means is configured such that based on the tracked radio frequency bursts and/or the gradient magnetic field the at least one first circuit and/or the electronic system as a whole is directly and/or indirectly synchronized with the radio frequency bursts and/or the gradient magnetic field.

For instance, the electronic system may be a component and/or an integral part of a medical device. It is possible that the electronic system can be implemented in an already existing medical device for upgrade purposes. This electronic system may be applicable to implantable devices for which it is imperative that some electronic circuits remain active even during an MRI scan. Examples of this can be power circuits, such as capacitive charge pumps.

The medical device may be e.g. embodied as an implantable, active medical device. In particular, the medical device can be e.g. a deep brain stimulation system. Such a deep brain stimulation system may be an implantable neurostimulation device, which can be used to treat acute or chronic neurological conditions. Deep brain stimulation (DBS), the mild electrical stimulation of sub-cortical structures, belongs to the category of implantable devices, and has been shown to be therapeutically effective for Parkinson's disease, Dystonia, and Tremor. New applications of DBS in the domain of psychiatric disorders (obsessive compulsive disorder, depression) are being researched and show promising results.

The magnetic resonance imaging apparatus, whose radio frequency bursts and/or a gradient magnetic field may be tracked, may be a magnetic resonance imaging apparatus, which is directly and/or indirectly influencing the electronic system.

According to the invention the advantage may be achieved that the electronics of e.g. an implant are configured such that it will not interfere with the MRI imaging making process without switching off the electronics of the implant. Consequently, it is not necessary to completely switch off the medical device.

It is possible that the at least one tracking means comprises at least one magnetic resonance imaging radio frequency burst detection circuit, which is configured such that radio frequency bursts and/or a gradient magnetic field of a magnetic resonance imaging apparatus are detectable by this magnetic resonance imaging radio frequency burst detection circuit.

The magnetic resonance imaging radio frequency burst detection circuit may be part of the synchronizing means. It is possible that the magnetic resonance imaging radio frequency burst detection circuit comprises a magnetic resonance imaging (MRI) radio frequency (RF) burst detector.

The MRI RF burst detector may be configured such that it outputs a signal/flag on its burst line to the at least one first circuit when an RF burst and/or gradient magnetic field is detected. This circuit can then be turned on as long as this flag is present. Of course, this circuit can also ignore this signal if it is not necessary for the circuit to be turned on.

Moreover, it is possible that the electronic system is configured such that the electronic system may operate in a normal operating mode and a magnetic resonance imaging scanning mode, whereby the electronic system further comprises at least one first circuit configured such that the at least one first circuit operates in the magnetic resonance imaging scanning mode during a magnetic resonance imaging scan, at least one switching means configured such that the switching means may switch the electronic system between the normal operating mode of the electronic system and the magnetic resonance imaging scanning mode of the electronic system.

The normal operating mode of the electronic system can be an operating mode, wherein all functions of the electronic system and also all functions of the medical device are available. The magnetic resonance imaging scanning mode of the electronic system may be an operating mode of the electronic system and the medical device, whereby only the necessary and essential functions of the medical device are available.

Preferably, it is possible that the electronic system further comprises at least one second circuit configured such that this second circuit is switched off completely in the magnetic resonance imaging scanning mode.

Moreover, it is also possible that the switching means is configured such that the switching means is connectable to an external system, whereby the external system forces the switching means to switch between the normal operating mode and the magnetic resonance imaging scanning mode, whereby the switching means is preferably equipped with radio frequency means and/or inductive communication means.

Additionally, it is possible that the electronic system further comprises at least one detection means, whereby the detection means is configured such that the presence of a magnetic resonance imaging scanner can be detected automatically. Thereby, the advantage is achieved that no manual switching operation has to be conducted to switch the electronic system between the normal operating mode of the electronic system and the magnetic resonance imaging scanning mode of the electronic system.

Furthermore, the switching means may be configured such that depending on a detection signal of the detection means the electronic system can be switched between normal operating mode and magnetic resonance imaging scanning mode.

In a preferred embodiment it is possible that the detection means is configured such that a magnetic resonance imaging detection signal is generated, when the static magnetic field of the magnetic resonance imaging machine is detected. Since any magnetic resonance imaging machine has a normally extremely strong static magnetic field, the presence of this field can be detected by the detection means and thus the electronic system is able to detect a magnetic resonance imaging machine.

Additionally, it is possible that the detection means advantageously comprises a Hall sensor means. A sensor means, which may be e.g. embodied as a simple hall sensor, allows an accurate and secure detection of a normally extremely strong static magnetic field of the magnetic resonance imaging machine. In particular, the static magnetic field of the magnetic resonance imaging machine may have a static magnetic field of about 1.0 T to 3.0 T. But it is also possible that the static magnetic field is above 3.0 T.

It is possible that the detection means may be configured such that a detection signal is generated when the first radio frequency burst of the MRI apparatus and/or the gradient magnetic field as flag to switch to magnetic resonance imaging mode is detected.

Preferably, it is possible that the at least one magnetic resonance imaging radio frequency burst detection circuit is embodied as a magnetic resonance imaging radio frequency burst and/or gradient magnetic field detector, whereby the magnetic resonance imaging radio frequency burst and/or gradient magnetic field detector comprises at least a magnetic antenna and/or a radio frequency detector.

Moreover, the switching means may comprise a switching back means, whereby the switching back means comprises at least one first time out means and/or at least one second time out means.

The first time out means may be configured such that the first time out means is capable to provide the switching means with at least one signal to switch the electronic system to the magnetic resonance imaging scanning mode for a time out phase and to switch the electronic system back to normal operating mode after the time out phase.

The second time out means may be configured such that the second time out means is triggered by a detected radio frequency burst and/or gradient magnetic field by the magnetic resonance imaging radio frequency burst detection circuit and that the second time out means thereafter provides the switching means with at least one signal to switch to magnetic resonance imaging scanning mode for a time out phase and switch back to normal mode after a time out phase.

It is also possible that the switching back means is configured such that it can be manually activated such that a switch back to normal mode can be conducted.

Moreover, the electronic system may further comprise at least one first radio frequency burst and/or gradient magnetic field time out timer means, which is configured such that it is directly and/or indirectly assured by the radio frequency burst and/or gradient magnetic field time out timer means that circuit activity of the first circuit is switched off when or before the radio frequency burst and/or gradient magnetic field ends.

The at least one first radio frequency burst and/or gradient magnetic field time out timer means may be embodied as or comprise at least one radio frequency (RF) burst and/or gradient magnetic field time out timer.

The RF burst and/or gradient magnetic field time out timer may be e.g. notified by the magnetic resonance imaging radio frequency burst detection circuit, via a signal on its output burst line, that an RF burst and/or gradient magnetic field is being detected, and therefore, can measure the duration of a detected RF burst and/or gradient magnetic field pulse.

The time out timer can be set to this measured duration, or any smaller fraction of it, before the subsequent RF burst and/or gradient magnetic field is detected. Thus when time out timer is triggered by the magnetic resonance imaging radio frequency burst detection circuit when the subsequent RF pulse and/or gradient magnetic field is detected, it can be assured that the first circuit is completely switched off when or before this subsequent RF burst and/or gradient magnetic field pulse ends.

For instance, one can either keep measuring the duration of every next RF burst and/or gradient magnetic field pulse as timer input for the subsequent RF pulse and/or gradient magnetic field pulse, measure once and use this value for all next detected RF bursts and/or gradient magnetic field pulses (i.e. for the whole duration that electronic system is in MRI mode), or finally, one could also opt to pre-program the time out phase duration and use this every time an RF burst and/or gradient magnetic field is detected by the magnetic resonance imaging radio frequency burst detection circuit.

For the first detected RF burst and/or gradient magnetic field pulse, the at least one first RF burst and/or gradient magnetic field time out timer may simply pass the signal on the burst line of the at least one magnetic resonance imaging radio frequency burst detection circuit to the first circuit directly as if the timer were not present.

Alternatively and/or additionally, it is possible that the electronic system further comprises at least one second radio frequency burst and/or gradient magnetic field time out timer means, which is configured such that the presence of an externally applied signal is directly and/or indirectly detected by the second frequency burst and/or gradient magnetic field time out timer means and that by this the second frequency burst and/or gradient magnetic field time out timer means is controlled and that circuit activity of the first circuit is switched off when the time out signal of the second frequency burst and/or gradient magnetic field time out timer means is generated, whereby especially the time out signal is generated before the radio frequency burst and/or gradient magnetic field has decayed away.

The second radio frequency burst and/or gradient magnetic field time out timer means may be e.g. equipped with an RF antenna. In this way, the second radio frequency burst and/or gradient magnetic field time out timer means might be notified, or once in a while notified, if this second time out timer means has an internal clock for synchronization, when the MRI machine is transmitting an RF pulse and/or gradient magnetic field.

The second time out timer may use the externally applied signal in the same way the first time out timer applies the detection signal from the magnetic resonance imaging radio frequency burst detection circuit.

The externally applied signal may also be used to control the circuit activity of the first circuit directly e.g. the externally applied signal may be used to switch the first circuit on and off directly instead of via the second time out timer.

Furthermore, the present invention relates to a medical system with the features of claim 15. Accordingly, a medical system comprises at least one electronic system according to one of the preceding claims.

For example, the medical system may be a system for neuro applications. Preferably, the medical system may be a system for brain applications, e.g. a deep brain stimulation system.

By this, e.g. the advantage can be achieved that the deep brain stimulation system must not be completely switched off during a magnetic resonance imaging process. Additionally, the magnetic resonance imaging process is not affected by the deep brain stimulation system e.g. due to interferences and thus magnetic resonance imaging can be conducted with high quality.

The electronic system is preferably an integral part and/or component of the deep brain stimulation system and can also be a component of the parts of the deep brain stimulation system, which is implanted into the patient.

Further details and advantages of the present invention shall be described hereinafter with respect to the drawings:

Fig. 1:     a schematical drawing of a typical radio frequency (RF) waveform;

Fig. 2a:    a schematical drawing of a first embodiment of the electronic system according to the present invention;

Fig. 2b:    a schematical drawing of a modification of the first embodiment of the electronic system according to the present invention;

Fig. 3:     a schematical drawing of a second embodiment of the electronic system according to the present invention;

Fig. 4:     a schematical drawing of a third embodiment of the electronic system according to the present invention;

Fig. 5: a schematical drawing of a fourth embodiment of the electronic system according to the present invention;

Fig. 6: a schematical drawing of a fifth embodiment of the electronic system according to the present invention;

Fig. 7: a schematical drawing of a sixth embodiment of the electronic system according to the present invention;

Fig. 8: a schematical drawing of a seventh embodiment of the electronic system according to the present invention;

Fig. 9: a schematical drawing of an eighth embodiment of the electronic system according to the present invention;

Fig. 10: a schematical drawing of a ninth embodiment of the electronic system according to the present invention;

Fig. 11: a schematical drawing of a tenth embodiment of the electronic system according to the present invention;

Fig. 12: a schematical drawing of a typical worst-case gradient waveform for a 1.0 T MRI system;

Fig. 13: a schematical drawing of a neurostimulation system for deep brain stimulation (DBS);

Fig. 14: a further schematical drawing of a probe neurostimulation system for deep brain stimulation (DBS) and its components; and

Fig. 15: a schematical drawing of a probe system according to the present invention.

Magnetic resonance imaging scanners perform their imaging in a periodic fashion. A strong static magnetic field aligns the spins in the nuclei of the investigated tissue of the scanned person. A radio frequency (RF) field is superimposed to flip the spins that were aligned by the static magnetic field. This RF field is composed of a short, very powerful burst absorbed by the tissue of the scanned person. For the excitation of hydrogen nuclei (and thus e.g. also water), the frequency is 42.57 MHz/T or approximately 64 MHz for a 1.5 T system, approximately 128 MHz for a 3.0 T system. After the RF burst, a magnetic resonance imaging (MRI) machine switches to "listening" mode to detect the radio waves emitted by the tissue as the (hydrogen) nuclei revert to their original spin state. The frequency of these very weak radio waves is the same as the frequency of the initially superimposed RF field (i.e. 64 MHZ for a 1.5 T scanner). This is the reason why an MRI machine is so extremely sensitive for any emitted electromagnetic radiation, for example by an implant, in the RF band of the scanner.

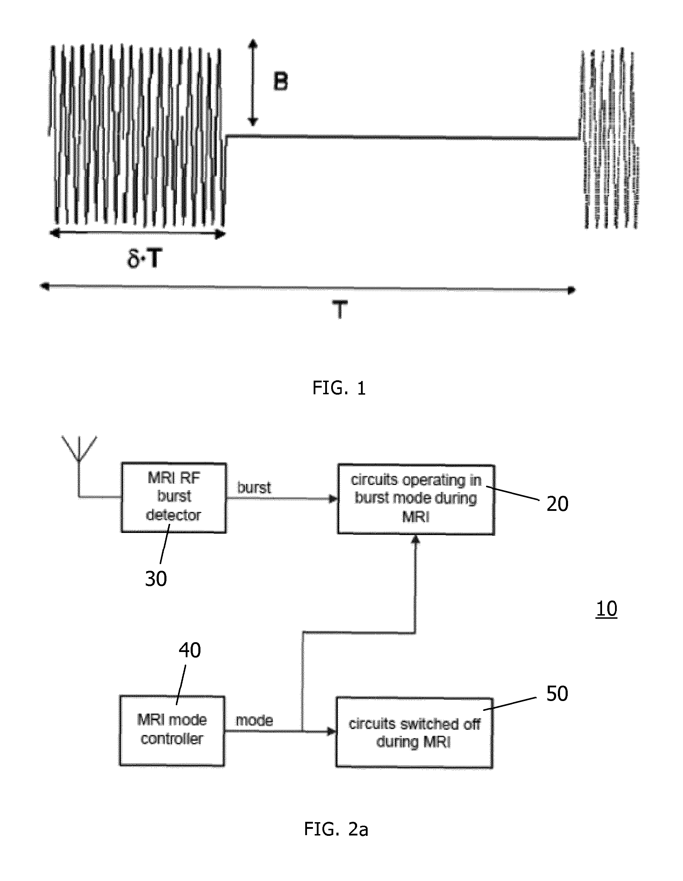

Figure 1 depicts an RF burst. The typical RF waveform is showing its high frequency sinusoidal burst character with a duration $\delta T$, the period T and the magnetic filed strength amplitude B.

The total RF cycle time is T, which is typically in the order of 10ms. The RF burst itself typically takes less than 5 % (i.e. < 0.05 typically) of the total time T. In the remaining time, the MRI scanner is in listening mode.

Figure 2a shows schematically a first embodiment of the electronic system 10 according to the present invention.

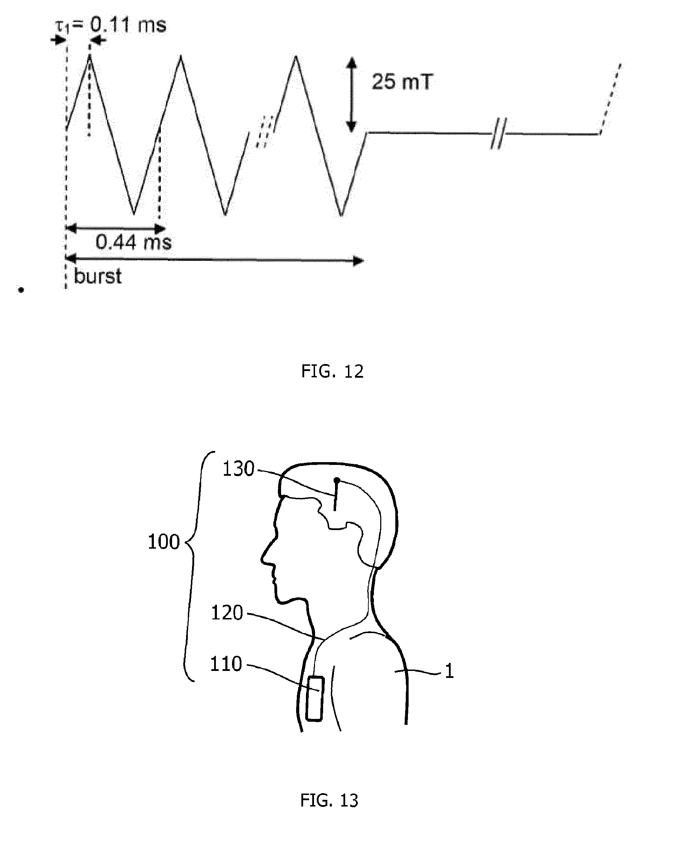

The electronic system 10 is an electronic system 10 for a medical device, e.g. for a deep brain stimulation system 100 (see Figures 13 to 15). The electronic system 10 is configured such that the electronic system 10 may operate in a normal operating mode and a magnetic resonance imaging scanning mode, whereby the electronic system 10 further comprises at least one first circuit 20 configured such that the at least one first circuit 20 operates in a duty-cycle mode, during a magnetic resonance imaging scan.

Moreover, the electronic system 10 has at least one magnetic resonance imaging radio frequency (MRI RF) burst detection circuit 30 and at least one switching means 40 configured such that the switching means may switch the electronic system 10 between the normal operating mode of the electronic system 10 and the magnetic resonance imaging scanning mode of the electronic system 10. The switching means 40 is embodied as a MRI mode controller 40.

The MRI mode controller 40 puts the electronic system 10 in its "MRI" mode which implies that some parts are switched off completely, while other parts are turned on periodically every time the MRI machine sends out an RF burst.

The switching means 40, respectively the MRI mode controller 40 may be configured such that the switching means 40 is connectable to an external system, whereby the external system forces the switching means 40 to switch between the normal operating mode and the magnetic resonance imaging scanning mode, whereby the switching means 40 is preferably equipped with radio frequency means and/or inductive communication means.

The electronic system 10 further comprises at least one second circuit 50 configured such that this second circuit 50 is switched off completely during a magnetic resonance imaging scan.

The MRI RF burst detection circuit 30 is embodied as MRI RF burst detector 30 and by means of the MRI RF burst detector 30 the occurrence of an RF burst can easily be detected. It is assumed that the activity of circuit 20 during an RF burst has no effect on the quality of the MRI scan. Consequently, one can operate electronic circuits for some (essential) functions during MRI imaging if this circuit activity is synchronized with the emitted RF bursts of an MRI machine.

Therefore, it is possible with electronic system 10 shown in Figure 2a that some electronics 50 is switched off completely, and the remaining electronic circuits 20 operate in sync with the RF bursts of the MRI scanner so that the circuits 20 are only active at the moment the MRI scanner is not sensitive for the potential interference generated by the activity of the circuits 20. As synchronization signal for the circuits 20, the output signal burst of the RF detector 30 is applied.

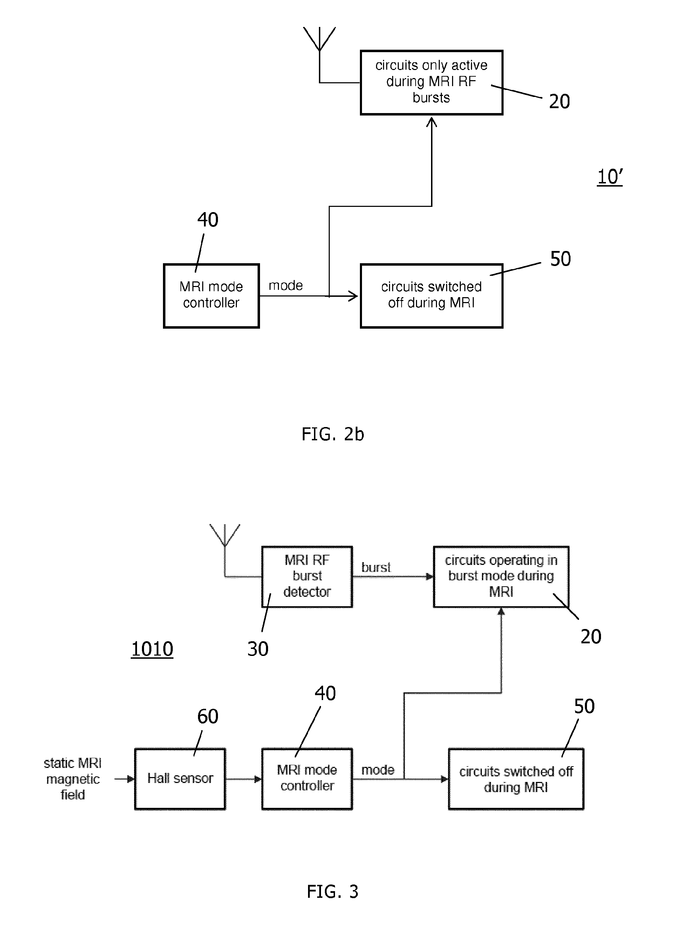

Figure 2b shows a modification of the first embodiment of the electronic system 10' according to the present invention. All components of the electronic system 10' are the same as of the electronic system 10 as shown in Figure 2a. As shown in Figure 2b, it is also possible, to provide the at least one circuit 20 with an external signal detecting means, e.g. an RF receiver, which is included into the circuit 20. So the circuit 20 may be directly controlled via an externally applied signal. For instance, the externally applied signal may be used to switch the first circuit on and off directly.

Figure 3 shows schematically a second embodiment of the electronic system 1010 according to the present invention, which comprises all elements of the first embodiment of the electronic system 10 shown in Figure 2a and which is equipped with further advantageous optional features.

The electronic system 1010 also comprises the first circuit 20, MRI RF burst detector 30, a MRI mode controller 40 as switching means 40 and second circuit 50.

Furthermore, the electronic system 1010 further comprises at least one detection means 60, whereby the detection means 60 is configured such that the presence of a magnetic resonance imaging scanner can be detected automatically. The detection means of the embodiment shown in Figure 3 is a Hall sensor 60, which generates a magnetic resonance imaging detection signal, when the static magnetic field of the magnetic resonance imaging machine is detected.

The switching means 40 is configured such that depending on a detection signal of the Hall sensor 60 the electronic system 1010 may be switched between normal operating mode and magnetic resonance imaging scanning mode automatically. The sensitivity of the Hall sensor 60 is configured such that the MRI mode controller 40 is not falsely triggered by other external magnetic fields than the static MRI field under normal operating conditions (i.e. daily life).

In both embodiments shown in Figure 2a and 3 it is possible that the switching means 40 comprises a switching back means, whereby the switching back means comprises at least one first time out means, whereby the first time out means is configured such that the first time out means is capable to provide the switching means 40 with at least one signal to switch the electronic system 10 or 1010 to the magnetic resonance imaging scanning mode for a time out phase and to switch the electronic system 10 or 1010 back to normal operating mode after the time out phase automatically.

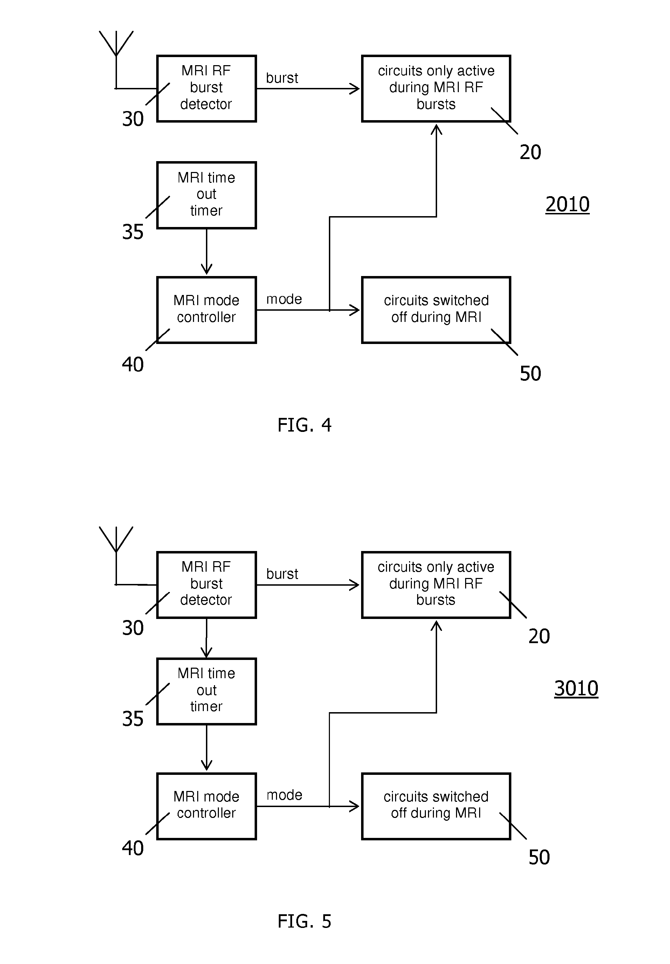

Figure 4 shows schematically a third embodiment of the electronic system 2010 according to the present invention, which comprises all elements of the first embodiment of the electronic system 10 shown in Figure 2a and which is equipped with further advantageous optional features.

The electronic system 2010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is embodied as a stand alone MRI time out timer and provides the MRI mode controller 40 with at least one signal to switch to magnetic resonance imaging scanning mode for a time out phase and switch back to normal mode after a time out phase automatically.

Figure 5 shows schematically a fourth embodiment of the electronic system 3010 according to the present invention, which comprises all elements of the first embodiment of the electronic system 10 shown in Figure 2a and which is equipped with further advantageous optional features.

The electronic system 10 also comprises the first circuit 20, MRI RF burst detector 30, a MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

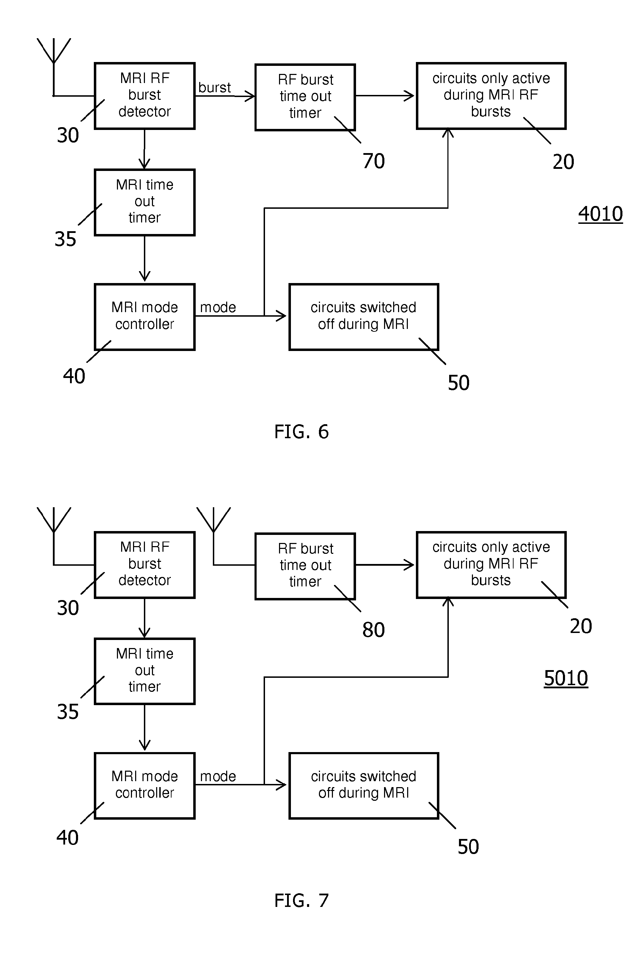

Figure 6 shows schematically a fifth embodiment of the electronic system 4010 according to the present invention, which comprises all elements of the first embodiment of the electronic system 10 shown in Figure 2a and which is equipped with further advantageous optional features.

The electronic system 4010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

Moreover, the electronic system 4010 further comprises at least one first radio frequency burst and/or gradient magnetic field time out timer means 70, which is configured such that it is directly and/or indirectly assured by the radio frequency burst and/or gradient magnetic field time out timer means 70 that circuit activity of the first circuit 20 is switched off when or before the radio frequency burst and/or gradient magnetic field ends.

The at least one first radio frequency burst and/or gradient magnetic field time out timer means 70 may be embodied as or comprise at least one radio frequency (RF) burst and/or gradient magnetic field time out timer.

The RF burst and/or gradient magnetic field time out timer may be e.g. notified by the magnetic resonance imaging radio frequency burst detection circuit 30, via a signal on its output burst line, that an RF burst and/or gradient magnetic field is being detected, and therefore, can measure the duration of a detected RF burst and/or gradient magnetic field pulse.

The time out timer 70 can be set to this measured duration, or any smaller fraction of it, before the subsequent RF burst and/or gradient magnetic field is detected. Thus when time out timer 70 is triggered by the magnetic resonance imaging radio frequency burst detection circuit 30 when the subsequent RF pulse and/or gradient magnetic field is detected, it can be assured that first circuit 20 is completely switched off when or before this subsequent RF burst and/or gradient magnetic field pulse ends.

For instance, one can either keep measuring the duration of every next RF burst and/or gradient magnetic field pulse as timer input for the subsequent RF pulse and/or gradient magnetic field pulse, measure once and use this value for all next detected RF bursts and/or gradient magnetic field pulses (i.e. for the whole duration that electronic system 4010 is in MRI mode), or finally, one could also opt to pre-program the time out phase duration and use this every time an RF burst and/or gradient magnetic field is detected by the magnetic resonance imaging radio frequency burst detection circuit 30.

For the first detected RF burst and/or gradient magnetic field pulse, the at least one first RF burst and/or gradient magnetic field time out timer may simply pass the signal on the burst line of the at least one magnetic resonance imaging radio frequency burst detection circuit to the first circuit directly as if the timer were not present.

Figure 7 shows schematically a sixth embodiment of the electronic system 5010 according to the present invention, which comprises all elements of the first embodiment of the electronic system 10 shown in Figure 2a and which is equipped with further advantageous optional features.

The electronic system 5010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

As shown in Figure 7, it is possible that the electronic system 5010 further comprises at least one second radio frequency burst and/or gradient magnetic field time out timer means 80, which is configured such that the presence of an externally applied signal is directly and/or indirectly detected by the second frequency burst and/or gradient magnetic field time out timer means 80 and that by this the second frequency burst and/or gradient magnetic field time out timer means 80 is controlled and that circuit activity of the first circuit 20 is switched off when the time out signal of the second frequency burst and/or gradient magnetic field time out timer means 80 is generated. In any case in this embodiment, the time out signal is generated before the radio frequency burst and/or gradient field has decayed away.

The second radio frequency burst and/or gradient magnetic field time out timer means may be e.g. equipped with an RF antenna. In this way, the second radio frequency burst and/or gradient magnetic field time out timer means 80 might be notified, or once in a while notified, if this second time out timer means 80 has an internal clock for synchronization, when the MRI machine is transmitting an RF pulse and/or gradient magnetic field.

The second time out timer may use the externally applied signal in the same way the first time out timer applies the detection signal from the magnetic resonance imaging radio frequency burst detection circuit.

The externally applied signal may also be used to control the circuit activity of the first circuit directly e.g. the externally applied signal may be used to switch the first circuit on and off directly instead of via the second time out timer.

The option to use a time out timer 35 as shown in the embodiment of Figure 4 to 7 can be extended by a means to put the electronics back into normal operating mode manually, immediately after the person has undergone an MRI scan.

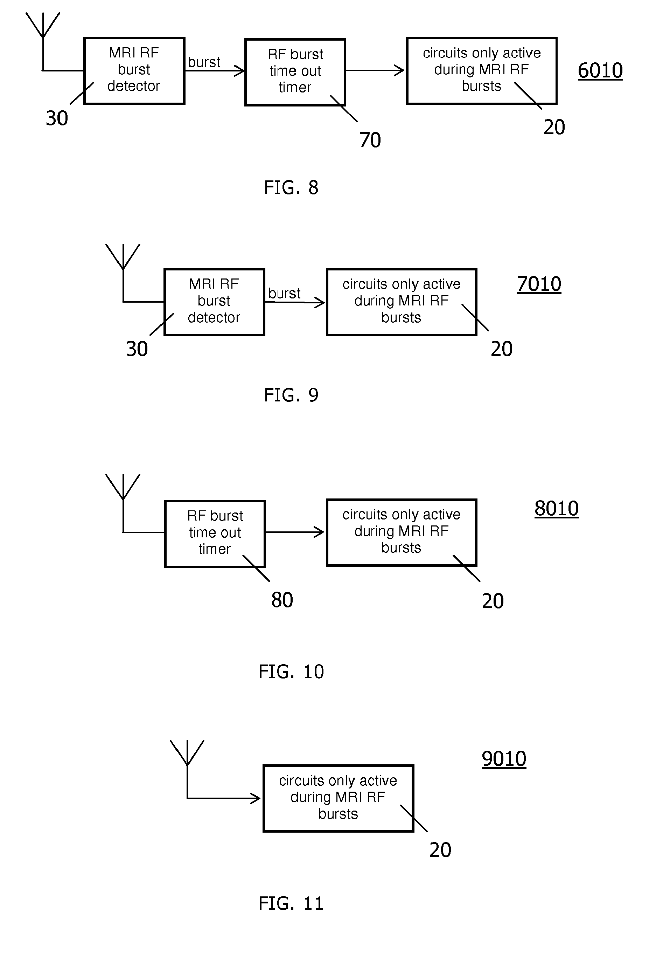

Figures 8 to 11 show single mode systems. These systems are "MRI only mode" systems.

The first option is shown in Figure 8, which shows the electronic system 6010. The electronic system 6010 comprises an MRI RF burst detector 30, an RF burst time out timer 70 and at least one first circuit 20. Likewise the embodiment shown in Figure 2a, the electronic circuit 20 operates in sync with the RF bursts of the MRI scanner so that the circuit 20 (or circuits 20) are only active at the moment the MRI scanner is not sensitive for the potential interference generated by the activity of the circuits 20. As synchronization signal for the circuits 20, the output signal burst of the RF detector 30 is applied.

The RF burst time out timer 70 may be an RF burst and/or gradient magnetic field time out timer 70 which is notified by magnetic resonance imaging radio frequency burst detection circuit 30, via a signal on its output burst line, that an RF burst and/or gradient magnetic field is being detected, and therefore, can measure the duration of a detected RF burst and/or gradient magnetic field pulse.

The time out timer can be set to this measured duration, or any smaller fraction of it, before the subsequent RF burst and/or gradient magnetic field is detected. Thus when time out timer 70 is triggered by detection circuit 30 when the subsequent RF pulse and/or gradient magnetic field is detected, it can be assured that the first circuit 20 is completely switched off when or before this subsequent RF burst and/or gradient magnetic field pulse ends.

One can either keep measuring the duration of every next RF burst and/or gradient magnetic field pulse as timer input for the subsequent RF pulse and/or gradient magnetic field pulse, measure once and use this value for all next detected RF bursts and/or gradient magnetic field pulses (i.e. for the whole duration that system 6010 is in MRI mode), or finally, one could also opt to pre-program the time out phase duration and use this every time an RF burst and/or gradient magnetic field is detected by circuit 30.

For the first detected RF burst and/or gradient magnetic field pulse, the at least one first RF burst and/or gradient magnetic field time out timer may simply pass the signal on the burst line of the at least one magnetic resonance imaging radio frequency burst detection circuit to the first circuit directly as if the timer were not present.

The second option is shown in Figure 9, which shows the electronic system 7010. The electronic system 7010 comprises an MRI RF burst detector 30 and at least one first circuit 20. Likewise the embodiment shown in Figure 2a or 8, the electronic circuit 20 operates in sync with the RF bursts of the MRI scanner so that the circuit 20 (or circuits 20) are only active at the moment the MRI scanner is not sensitive for the potential interference generated by the activity of the circuits 20. As synchronization signal for the circuits 20, the output signal burst of the RF detector 30 is applied.

The third option is shown in Figure 10, which shows the electronic system 8010. The electronic system 8010 comprises at least one second radio frequency burst and/or gradient magnetic field time out timer means 80 and at least one first circuit 20. Likewise the embodiment shown in Figure 7, at least one second radio frequency burst and/or gradient magnetic field time out timer means 80, which is configured such that the presence of an externally applied signal is directly and/or indirectly detected by the second frequency burst and/or gradient magnetic field time out timer means 80 and that by this the second frequency burst and/or gradient magnetic field time out timer means 80 is controlled and that circuit activity of the first circuit 20 is switched off when the time out signal of the second frequency burst and/or gradient magnetic field time out timer means 80 is generated. In any case in this embodiment, the time out signal is generated before the radio frequency burst and/or gradient field has decayed away.

The fourth option is shown in Figure 11, which shows the electronic system 9010. The electronic system 9010 comprises at least one first circuit 20, which is modified. Likewise the embodiment shown in Figure 2b, this modified circuit 20 is equipped with an external signal detecting means, e.g. an RF receiver, which is included into the circuit 20. So the circuit 20 may be directly controlled via an externally applied signal. For instance, the externally applied signal may be used to switch the first circuit on and off directly.

In all embodiments described above and shown in Figures 2 to 9 it is possible that the MRI RF burst detector 30 can consist of a magnetic antenna (i.e. coil) and an RF detector. The RF frequency $f_{RF}$ of the MRI scanner is related to the field strength $B_{MRI}$ of the static field:

$$f_{RF}(B_{MRI}) = 42.58 * B_{MRI} * \frac{MHz}{T}$$

The maximum field strength $B_{max}$ of the RF field varies spatially and is also related to the type of scanner. In the centre of an MRI scanner, the amplitude can be up to 27 $\mu$T for 1.5 T systems and up to 13 $\mu$T in 3 T MRI machines. Close to the rings of the RF coil, the amplitude of the RF field is the highest and can be up to 45 $\mu$T. For this maximum amplitude $B_{max}$ of

$$B_{max} = 45 \mu T$$

the maximum dB/dt is

$$\frac{dB}{dt}_{max} = 2 * \pi * B_{max} * f_{RF}$$

which for a 1.5 T scanner translates into

$$\frac{dB}{dt_{\max,1.5T}} = 1.80588 * 10^4 \frac{kg}{A * s^3}$$

and which for a 3.0 T scanner translates into

$$\frac{dB}{dt_{\max,3.0T}} = 3.61176 * 10^4 \frac{kg}{A * s^3}$$

The maximum induced voltage $V_{\max}$ in a single turn coil with area A is

$$V_{\max} = A * \frac{dB}{dT_{\max}}$$

and with only a surface area of 1 cm$^2$ one gets

$$V_{\max,1.5T} = 1.8\ V$$

and

$$V_{\max,3.0T} = 3.6\ V$$

These signal levels can easily be detected by a simple rectification circuit and level detector, even when they are an order of magnitude smaller (i.e. if the electronics is not located near the "hot spot" RF coils). The detector 30 can be designed without disturbing the picture making process during the "listening" mode of the MRI scanner.

False triggers of the burst detector can be prevented by not only setting the detector threshold level appropriately (optionally depending of the type of MRI machine and/or adaptively) but also by using a tuned coil circuit (i.e. filtering at the RF frequency of the MRI machine at hand).

Gradient fields are applied for spatial encoding of the image. Typically, their frequency range is between 0 Hz and 5 kHz. The waveforms can have very different shapes and are certainly not always sinusoidal. The amplitudes of the gradient fields vary strongly in the patient space inside the MRI scanner. The amplitudes are limited by requirements on nerve stimulation.

As an illustration for the case of a 1.0 T "open" MRI machine, the strongest appearing gradient field is shown in Figure 12. In general, the amplitude of a gradient field is typically 3 orders of magnitude larger ($\mu$T vs.

mT) than the RF field strength, while their frequency is about 4 to 5 orders smaller (64 MHz vs. 0-5 kHz). Therefore, the induced voltages, proportional to field strength and frequency, are 1 to 2 orders smaller than for the RF fields.

Theoretically, one could also use the gradient fields instead of the RF bursts to switch (part of) the electronics on periodically during MRI scanning but the gradient field detector must have a much higher sensitivity due to the much lower induced voltages.

However, more importantly, the gradient field burst may also appear during the listening mode of an MRI scanner, depending on the chosen settings during scanning, which implies that the switched electronics might interfere with the picture making process. Therefore, the gradient fields are not suitable for detection purposes in general.

The whole electronics of the deep brain stimulation (DBS) system 100 may be formed by the electronic system 10 as described above. Such a DBS system 100 may be embodied at least partially as shown in Figures 13 to 15 and as described below.

A possible embodiment of a neurostimulation system 100 for deep brain stimulation (DBS) is shown in Figure 13. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

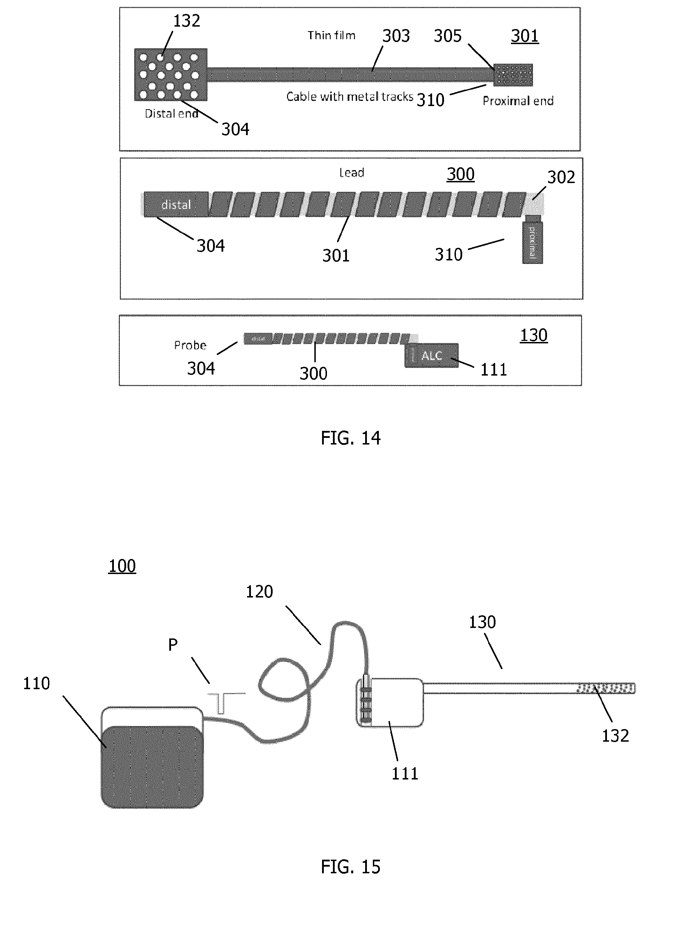

Figure 14 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrodes 132 to a designated proximal contact 305.

Figure 15 shows schematically and in greater detail an embodiment of a system 100 for brain applications, here for neurostimulation and/or neurorecording as a deep brain stimulation system 100 as shown in Figures 13 and 14. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

**Claims**

1. An electronic system (10, 10', 1010, 2010, 3010, 4010, 5010, 6010, 7010, 8010, 9010), especially an electronic system (10, 10', 1010, 2010, 3010, 4010, 5010, 6010, 7010, 8010, 9010) for a medical device, comprising at least one first circuit (20), at least one tracking means, which is configured such that radio frequency bursts and/or a gradient magnetic field of a magnetic resonance imaging apparatus can be tracked, and at least one synchronizing means, whereby the synchronizing means is configured such that based on the tracked radio frequency bursts and/or the gradient magnetic field the at least one first circuit (20) and/or the electronic system (10, 10', 1010, 2010, 3010, 4010, 5010, 6010, 7010, 8010, 9010) as a whole is directly and/or indirectly synchronized with the radio frequency bursts and/or the gradient magnetic field.

2. The electronic system (10, 1010, 2010, 3010, 4010, 5010, 6010, 7010) according to claim 1, **characterized in that** the at least one tracking means comprises at least one magnetic resonance imaging radio frequency burst detection circuit (30), which is configured such that radio frequency bursts and/or a gradient magnetic field of a magnetic resonance imaging apparatus are detectable by magnetic resonance imaging radio frequency burst detection circuit (30).

3. The electronic system (10, 1010, 2010, 3010, 4010, 5010) according to claim 1 or 2, **characterized in that** electronic system (10, 1010, 2010, 3010, 4010, 5010) is configured such that the electronic system (10, 1010, 2010, 3010, 4010, 5010) may operate in a normal operating mode and a magnetic resonance imaging scanning mode, whereby the electronic system (10, 1010, 2010, 3010, 4010, 5010) further comprises at least one first circuit (20) configured such that the at least one first circuit (20) operates in the magnetic resonance imaging scanning mode during a magnetic resonance imaging scan, at least one switching means (40) configured such that the switching means (40) may switch the electronic system (10, 1010, 2010, 3010, 4010, 5010) between the normal operating mode of the electronic sytem (10, 1010, 2010, 3010, 4010, 5010) and the magnetic resonance imaging scanning mode of the electronic system (10, 1010, 2010, 3010, 4010, 5010).

4. The electronic system (10, 1010, 2010, 3010, 4010, 5010) according to claim 3, **characterized in that** the electronic system (10, 1010, 2010, 3010, 4010, 5010) further comprises at least one second circuit (50) configured such that this second circuit (50) is switched off completely in the magnetic resonance imaging scanning mode and/or that the switching means (40) is configured such that the switching means (40) is connectable to an external system, whereby the external system forces the switching means (40) to switch between the normal operating mode and the magnetic resonance imaging scanning mode, whereby the switching means (40) is preferably equipped with radio frequency means and/or inductive communication means.

5. The electronic system (1010) according to any of the preceding claims, **characterized in that** the electronic system (1010) further comprises at least one detection means (60), whereby the detection means (60) is configured such that the presence of a magnetic resonance imaging apparatus can be detected automatically.

6. The electronic system (1010) according to claim 5, **characterized in that** the switching means (40) is configured such that depending on a detection signal of the detection means (60) the electronic system (1010) can be switched between normal operating mode and magnetic resonance imaging scanning mode.

7. The electronic system (1010) according to claim 5 or 6, **characterized in that** the detection means (60) is configured such that a magnetic resonance imaging detection signal is generated, when the static magnetic field of the magnetic resonance imaging machine is detected.

8. The electronic system (1010) according to any of claims 5 to 7, **characterized in that** the detection means (60) comprises a Hall sensor means.

9. The electronic system (1010) according to any of claim 5 to 8,
**characterized in that**
the detection means (60) is configured such that a detection signal is generated when the first radio frequency burst and/or the gradient magnetic field as flag to switch to magnetic resonance imaging mode is detected.

10. The electronic system (10, 1010, 2010, 3010, 4010, 5010, 6010, 7010) according to any of claims 2 to 9,
**characterized in that**
the at least one magnetic resonance imaging radio frequency burst detection circuit (30) is embodied as a magnetic resonance imaging radio frequency burst and/or gradient magnetic field detector, whereby the magnetic resonance imaging radio frequency burst and/or gradient magnetic field detector comprises at least a magnetic antenna and/or a radio frequency detector.

11. The electronic system (2010, 3010, 4010, 5010) according to any of claims 3 to 10,
**characterized in that**
the switching means (40) comprises a switching back means, whereby the switching back means comprises at least one first time out means and/or at least one second time out means (35), whereby the first time out means is configured such that the first time out means is capable to provide the switching means (40) with at least one signal to switch the electronic system (2010, 3010, 4010, 5010) to the magnetic resonance imaging scanning mode for a time out phase and to switch the electronic system (2010, 3010, 4010, 5010) back to normal operating mode after the time out phase and/or whereby the second time out means (35) is configured such that the second time out means (35) is triggered by a detected radio frequency burst and/or gradient magnetic field by the magnetic resonance imaging radio frequency burst detection circuit (30) and that the second time out means (35) thereafter provides the switching means (40) with at least one signal to switch to magnetic resonance imaging scanning mode for a time out phase and switch back to normal mode after a time out phase.

12. The electronic system (2010, 3010, 4010, 5010) according to claim 11,
**characterized in that**
the switching back means is configured such that it can be manually activated such that a switch back to normal mode can be conducted.

13. The electronic system (4010, 6010) according to any of the preceding claims,
**characterized in that**
the electronic system (4010, 6010) further comprises at least one first radio frequency burst and/or gradient magnetic field time out timer means (70), which is configured such that it is directly and/or indirectly assured by the radio frequency burst and/or gradient magnetic field time out timer means (70) that circuit activity of the first circuit (20) is switched off when or before the radio frequency burst and/or gradient magnetic field ends.

14. The electronic system (5010, 8010) according to any of the preceding claims,
**characterized in that**
the electronic system (5010, 8010) further comprises at least one second radio frequency burst and/or gradient magnetic field time out timer means (80), which is configured such that the presence of an externally applied signal is directly and/or indirectly detected by the second frequency burst and/or gradient magnetic field time out timer means (80) and that by this the second frequency burst and/or gradient magnetic field time out timer means (80) is controlled and that circuit activity of the first circuit (20) is switched off when the time out signal of the second frequency burst and/or gradient magnetic field time out timer means (80) is generated, whereby especially the time out signal is generated before the radio frequency burst and/or gradient magnetic field has decayed away.

15. A medical system, especially a system for neuro applications, preferably a system for brain applications, further preferably a deep brain stimulation system (100), comprising at least one electronic system (10, 1010, 2010, 3010, 4010, 5010, 6010, 7010) according to one of the preceding claims.

FIG. 1

FIG. 2a

circuits only active during MRI RF bursts

20

10'

40

MRI mode controller

mode

circuits switched off during MRI

50

FIG. 2b

MRI RF burst detector

burst

circuits operating in burst mode during MRI

1010

30

20

60

40

50

static MRI magnetic field

Hall sensor

MRI mode controller

mode

circuits switched off during MRI

FIG. 3

FIG. 4

FIG. 5

MRI RF burst detector
30

burst

RF burst time out timer
70

circuits only active during MRI RF bursts
20

4010

MRI time out timer
35

MRI mode controller
40

mode

circuits switched off during MRI
50

FIG. 6

MRI RF burst detector
30

RF burst time out timer
80

circuits only active during MRI RF bursts
20

5010

MRI time out timer
35

MRI mode controller
40

mode

circuits switched off during MRI
50

FIG. 7

MRI RF burst detector 30 → burst → RF burst time out timer 70 → circuits only active during MRI RF bursts 20    6010

FIG. 8

MRI RF burst detector 30 → burst → circuits only active during MRI RF bursts 20    7010

FIG. 9

RF burst time out timer 80 → circuits only active during MRI RF bursts 20    8010

FIG. 10

circuits only active during MRI RF bursts 20    9010

FIG. 11

$\tau_1$ = 0.11 ms

25 mT

0.44 ms

burst

FIG. 12

130

100

120

110

1

FIG. 13

132

Thin film    303    305    301

Cable with metal tracks 310    Proximal end

Distal end    304

Lead    300    302

distal

304    301    310    proximal

Probe    130

ALC

304    300    111

FIG. 14

100

120

P    130

110    111    132

FIG. 15

Europäisches
Patentamt

European
Patent Office

Office européen
des brevets

# EUROPEAN SEARCH REPORT

Application Number

EP 12 17 1506

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/088012 A1 (KROLL MARK W [US] ET AL) 6 May 2004 (2004-05-06) * paragraphs [0002], [0036], [0077] - [0125]; figures 1,2,5-9 * | 1-15 | INV. A61N1/37 A61N1/36 G01R33/28 |
| X | US 2011/156706 A1 (STUBBS SCOTT R [US] ET AL) 30 June 2011 (2011-06-30) * paragraphs [0051] - [0074]; figures 1-8 * | 1-12,15 | |
| X | US 2009/256574 A1 (WEISS STEFFEN [DE]) 15 October 2009 (2009-10-15) * paragraphs [0026], [0037] - [0053]; figure 1 * | 1-12,15 | |
| X | US 2007/238975 A1 (ZEIJLEMAKER VOLKERT A [NL]) 11 October 2007 (2007-10-11) * paragraphs [0011] - [0016], [0018]; figures 1-4,5A * | 1-15 | |
| X | US 2004/263172 A1 (GRAY ROBERT W [US] ET AL) 30 December 2004 (2004-12-30) * paragraphs [0094] - [0106], [0166], [0167]; figures 3-6 * | 1-10, 13-15 | TECHNICAL FIELDS SEARCHED (IPC) A61N G01R |
| X | US 2005/070787 A1 (ZEIJLEMAKER VOLKERT A [NL]) 31 March 2005 (2005-03-31) * paragraphs [0021] - [0044]; figures 1-4 * | 1-15 | |
| A | US 2011/196449 A1 (JENISON TROY A [US]) 11 August 2011 (2011-08-11) * paragraphs [0017] - [0048], [0053] - [0063], [0068]; figure 1 * | 3-9,11, 12,15 | |

-/--

The present search report has been drawn up for all claims

| Place of search | Date of completion of the search | Examiner |
|---|---|---|
| Munich | 31 January 2013 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 12 17 1506

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/063848 A1 (ST JUDE MEDICAL [SE]; SKOELDENGEN NIKLAS [SE]; ABRAHAMSON HANS [SE]; D) 3 June 2011 (2011-06-03)<br>* page 2, line 16 - page 3, line 2 *<br>* page 4, line 28 - page 5, line 12 *<br>* page 6, line 8 - page 6, line 17 *<br>* page 7, line 31 - page 16, line 9 *<br>* figures 1-4 *<br>----- | 1-10,12, 15 | |
| X | US 2005/070975 A1 (ZEIJLEMAKER VOLKERT A [NL] ET AL) 31 March 2005 (2005-03-31)<br>* paragraphs [0023] - [0057]; figures 1-6 *<br>----- | 1-15 | |
| X | US 2007/255332 A1 (CABELKA LONNY V [US] ET AL) 1 November 2007 (2007-11-01)<br>* paragraphs [0008] - [0012], [0020], [0024], [0025], [0028] - [0031], [0033] - [0037]; figures 1-4 *<br>----- | 1-12,14, 15 | |
| | | | TECHNICAL FIELDS SEARCHED (IPC) |

The present search report has been drawn up for all claims

| Place of search | Date of completion of the search | Examiner |
|---|---|---|
| Munich | 31 January 2013 | Lersch, Wilhelm |

EPO FORM 1503 03.82 (P04C01)

Europäisches
Patentamt
European
Patent Office
Office européen
des brevets

**Application Number**

EP 12 17 1506

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 12 17 1506

The Search Division considers that the present European patent application does not comply with the requirements of unity of invention and relates to several inventions or groups of inventions, namely:

1. claims: 1-12, 15

   Electronic system at least a first circuit of which is synchronized with the RF bursts and/or a gradient magnetic field of an MRI apparatus.
   ---

2. claims: 13, 14

   An RF burst and/or gradient magnetic field time out timer of the electronic system is configured such that circuit activity of the first circuit is switched off when or before the RF burst and/or gradient magnetic field ends.
   ---

## ANNEX TO THE EUROPEAN SEARCH REPORT
## ON EUROPEAN PATENT APPLICATION NO.

EP 12 17 1506

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2013

| Patent document cited in search report | | Publication date | Patent family member(s) | | Publication date |
|---|---|---|---|---|---|
| US 2004088012 | A1 | 06-05-2004 | NONE | | |
| US 2011156706 | A1 | 30-06-2011 | EP | 2519315 A1 | 07-11-2012 |
| | | | US | 2011156706 A1 | 30-06-2011 |
| | | | WO | 2011090594 A1 | 28-07-2011 |
| US 2009256574 | A1 | 15-10-2009 | CN | 101495881 A | 29-07-2009 |
| | | | EP | 2049911 A1 | 22-04-2009 |
| | | | US | 2009256574 A1 | 15-10-2009 |
| | | | WO | 2008015605 A1 | 07-02-2008 |
| US 2007238975 | A1 | 11-10-2007 | US | 2007238975 A1 | 11-10-2007 |
| | | | US | 2010152805 A1 | 17-06-2010 |
| | | | US | 2012010676 A1 | 12-01-2012 |
| | | | US | 2012265263 A1 | 18-10-2012 |
| | | | WO | 2007117835 A1 | 18-10-2007 |
| US 2004263172 | A1 | 30-12-2004 | CA | 2517771 A1 | 13-01-2005 |
| | | | EP | 1636612 A2 | 22-03-2006 |
| | | | JP | 2007524458 A | 30-08-2007 |
| | | | US | 2004263172 A1 | 30-12-2004 |
| | | | US | 2004263173 A1 | 30-12-2004 |
| | | | US | 2004263174 A1 | 30-12-2004 |
| | | | US | 2008058913 A1 | 06-03-2008 |
| | | | WO | 2005003790 A2 | 13-01-2005 |
| US 2005070787 | A1 | 31-03-2005 | NONE | | |
| US 2011196449 | A1 | 11-08-2011 | US | 2011196449 A1 | 11-08-2011 |
| | | | WO | 2011100239 A1 | 18-08-2011 |
| WO 2011063848 | A1 | 03-06-2011 | US | 2012229299 A1 | 13-09-2012 |
| | | | WO | 2011063848 A1 | 03-06-2011 |
| US 2005070975 | A1 | 31-03-2005 | AU | 2004278725 A1 | 14-04-2005 |
| | | | CA | 2540680 A1 | 14-04-2005 |
| | | | DE | 602004009704 T2 | 28-08-2008 |
| | | | EP | 1676142 A1 | 05-07-2006 |
| | | | US | 2005070975 A1 | 31-03-2005 |
| | | | US | 2010041978 A1 | 18-02-2010 |
| | | | WO | 2005033724 A1 | 14-04-2005 |
| US 2007255332 | A1 | 01-11-2007 | US | 2007255332 A1 | 01-11-2007 |
| | | | US | 2010198309 A1 | 05-08-2010 |
| | | | WO | 2007127705 A1 | 08-11-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7660620 B2

- US 20100106006 A1